# EUROPEAN PATENT APPLICATION

(11) **EP 3 925 616 A1**
(43) Date of publication of application: **22.12.2021**
(21) Application number: 20755567.3
(22) Date of filing: 10.02.2020
(51) Int. Cl.: A61K 38/55, A61P 7/02, A61P 13/12

(54) **METHOD FOR TREATING PREGANCY-INDUCED HYPERTENSION NEPHROSIS**

(30) Priority: 11.02.2019 US 201962803888 P; 19.03.2019 US 201962820459 P
(71) Applicant: Kyowa Kirin Co., Ltd., Tokyo 100-0004 (JP)
(72) Inventor: NAKAMURA Yoshihide, Tokyo 100-0004 (JP); MURAYAMA Takashi, Tokyo 100-0004 (JP); TAKAGI Kenichi, Tokyo 100-0004 (JP); SHIMAZAKI Ryutaro, Tokyo 100-0004 (JP); ENDO Yuichi, Tokyo 100-0004 (JP); KANDA Hironori, Tokyo 100-0004 (JP); MOTOYAMA Kayoko, Tokyo 100-0004 (JP); KATO Masaya, Tokyo 100-0004 (JP); ORIHARA Shunichiro, Tokyo 100-0004 (JP); TANAKA Tomoko, Tokyo 100-0004 (JP)
(74) Representative: Hoffmann Eitle
(86) International application number: PCT/JP2020/005130
(87) International publication number: WO 2020/166557

(57) **Abstract**

An object of the invention is to provide a method for treating preeclampsia, comprising administering antithrombin, or a novel composition for treating preeclampsia comprising antithrombin. The invention relates to a method for treating preeclampsia, comprising administering antithrombin to a preeclampsia patient having blood antithrombin activity of 100% or less, a composition for treating preeclampsia comprising antithrombin, which is administered to a preeclampsia patient having blood antithrombin activity of 100% or less and the like.

## Description

### Technical Field

The present invention relates to a method for treating preeclampsia, comprising administering antithrombin or a composition for treating preeclampsia comprising antithrombin.

### Background Art

Antithrombin is also called antithrombin III. Human antithrombin, which is naturally existing human antithrombin, is a glycoprotein composed of 432 amino acids having a molecular weight of about 59000 to 65000 and has three disulfide bonds (Cys8-Cys128, Cys21-Cys95 and Cys247-Cys430) in the molecule (NPL 1).

Human antithrombin is one of major coagulation inhibitors in the blood, forms complexes with Factor X, Factor XII, Factor IX, Factor XI or the like as well as with thrombin and thus inactivates these coagulation factors.

In Japan, formulations containing human antithrombin derived from human plasma (referred to as plasma-derived human antithrombin below) have been approved and used with a brand name of Neuart (registered trademark), Anthrobin (registered trademark) P or Kenketu Nonthron (registered trademark) in Japan for indications "disseminated intravascular coagulation (DIC) followed by reduction in antithrombin III" and "thrombophilia based on congenital antithrombin III deficiency". Moreover, portal vein thrombosis followed by reduction in antithrombin III has been also approved as an indication for Kenketu Nonthron (registered trademark).

A formulation containing antithrombin gamma, which is recombinant human antithrombin, has been approved and used as Acoalan (registered trademark) in Japan for indications "thrombophilia based on congenital antithrombin deficiency" and "disseminated intravascular coagulation (DIC) followed by reduction in antithrombin".

As another recombinant human antithrombin, recombinant human antithrombin in which fucose is not bound to N-acetylglucosamine in the reducing end of a complex type N-glycoside-linked sugar chain attached to human antithrombin has been known (PTL 1 and PTL 2).

Preeclampsia (referred to as PE below) is a disease type of hypertensive disorders of pregnancy (referred to as HDP below). HDP is defined as hypertension in pregnancy, and the disease types of HDP include gestational hypertension, superimposed preeclampsia and pregnancy with hypertension in addition to PE, and are classified into four disease types (NPL 2 and NPL 3).

A diagnosis of PE is made when hypertension and organ dysfunction of the mother or uteroplacental insufficiency are observed. It is reported that the prognoses of the mother and the child are relatively poor in PE and that the perinatal mortality rate, the incidence of fetal growth restriction, the incidence of newborns with birth weights less than 2500 g and the rate of neonatal NICU stay are all higher than those of normal pregnant women (NPL 4 and NPL 5).

Although the cause/pathological mechanism of HDP has not been elucidated, various reports have been made (NPL 6). Recently, the "two-stage disorder theory" is supported in PE.

Normally, influx from maternal vessels to the intervillous space starts at 10 to 12 weeks after implantation, causing an increase in oxygen partial pressure in the fetoplacental circulation. However, in PE, the increase in oxygen partial pressure does not occur in the fetoplacental circulation owing to spiral artery remodeling failure in the placenta, resulting in continued hypoxic conditions. Placental hypoxia/ischemia stimulates the production of soluble fms-like tyrosine kinase-1 (referred to as sFlt-1 below) and soluble endoglin (referred to as sEng below) in trophoblasic cells and suppresses placental growth factor (referred to as PlGF below) production. Therefore, a vicious circle of placental hypoxic conditions occurs from early pregnancy (First stage) (NPL 7).

The factors have transplacental capability. Because the factors cause the vicious circle of hypoxic conditions in the placental circulatory system and also move to the maternal circulatory system at the same time, the factors inhibit the functions of the vascular endothelial cells of the mother, resulting in the onset of hypertension and proteinuria (Second stage) (NPL 8 and NPL 9).

While the vascular endothelial dysfunction caused by the increase in sEng and sFlt-1 facilitates the production of thrombin, thrombin increases sFlt-1 (NPL 10). Thus, the vascular endothelial dysfunction may be further caused, and hypertension and proteinuria may be deteriorated. In addition, as a result of thrombin production, a decrease in antithrombin and an increase in thrombin/antithrombin complex are observed, resulting in thrombophilia. Inhibition of fibrinolysis becomes relatively high, and the state is considered as potential disseminated intravascular coagulation.

The radical cure for PE is to end the pregnancy. However, it is reported that the mortality rate of newborn is higher as the delivery becomes earlier (NPL 11). It is also reported that the shorter the gestational age at birth is, the higher the incidence rate of complications such as cerebral palsy is (NPL 12)

Accordingly, general procedures against PE are rest, dietetic treatment, medication to the mother and the like for the purpose of continuing the pregnancy as long as possible until the fetus becomes mature.

The drug therapy includes antihypertensive drugs, steroids, magnesium formulations and the like. An antihypertensive drug is mainly administered to serious hypertension. However, an extreme decrease in the pressure improves the maternal condition but adversely affects the fetus or the placental function, resulting in deterioration of the fetal condition (NPL 13). The deterioration in the fetal condition requires an early end of the pregnancy and also leads to an increase in the perinatal mortality rate.

Results of clinical trials of antithrombin for PE have been reported.

One thereof is phase III clinical trials conducted between July 1993 and August 1995 in which plasma-derived human antithrombin or albumin as a placebo was administered to pregnancy toxemia patients having total Gestosis Index (referred to as GI below) scores of 6 or more (corresponding to PE according to the current diagnostic standard). It is reported that, as a result of the trials, the human plasma-derived antithrombin significantly extended the gestational age as compared to the placebo (NPL 14 and NPL 15).

Trials in which recombinant antithrombin or a placebo was administered to early-onset preeclampsia patients have also been conducted. It is reported that, as a result of the trials, administration of recombinant antithrombin to early-onset preeclampsia patients was not relevant to extension of the pregnancy duration (NPL 16).

### Related Art

### Patent Document

PTL 1: WO2005/035563
PTL 2: WO2008/120801

### Non-Patent Document

NPL 1: Proc. Natl. Acad. Sci. USA. 80: 1845-1848, 1983
NPL 2: Mark A. Brown et. al. Hypertension. 72: 24-43, 2018
NPL 3: Japan Society for the Study of Hypertension in Pregnancy, New Definition and Classification of Hypertensive Disorders of Pregnancy, 2019
NPL 4: Barton et al. Am J Obstet Gynecol. 184(5):979-83, 2001
NPL 5: Xiong et al. J Reprod Med. 52(5):402-6, 2007
NPL 6: Contrib. Nephrol. 25: 108-110, 1981
NPL 7: Seki et al. Acta Obstet Gynecol Scand. 93(10): 959-64, 2014
NPL 8: Ahmad et al. Vasc Cell. 3(1):15, 2011
NPL 9: Walshe et al. PLoS One. 4(4):e5149, 2009
NPL 10: Lockwood et al. Am J Pathol. 170(4): 1398-1405, 2007
NPL 11: Tokumasu H et al. Pediatr Int. 58(7): 578-583, 2016
NPL 12: Nakanishi H et al. J Perinatol. 38(7): 917-928, 2018
NPL 13: Japan Society for the Study of Hypertension in Pregnancy, Practice Guide for Hypertensive Disorders of Pregnancy, Medical View Co., Ltd., 251p, 2015
NPL 14: Shinndan to Shinnyaku, Vol. 33, No. 1: 67-94, 1996
NPL 15: Thromb Haemost. 84: 583-590, 2000
NPL 16: American Journal of Obstetic. S559, LB02 Supplement to MONTH, 2017

### Summary of Invention

### Problems to Be Solved by the Invention

In treating PE, it is important to improve not only the maternal condition but also the fetal condition, prevent the progress of fetal growth restriction and prevent the birth of a low-birth-weight infant, but such a therapeutic method has not been established yet.

As explained above, it is reported in NPL 14 and NPL 15 that plasma-derived human antithrombin significantly extended the gestational ages of patients who would be diagnosed with PE according to the current diagnostic standard as compared to the placebo. However, the segment of PE patients on which human antithrombin has effects is not known.

An object of the invention is to provide a novel method for treating PE, comprising administering antithrombin or a novel composition for treating PE comprising antithrombin.

### Means for Solving the Problems

As a result of studies on the problems, the present inventors have found that human antithrombin extends the pregnancy duration of patients as compared to a placebo in a specific segment in PE. The invention which has been completed based on the findings is as follows.

1. A method for treating preeclampsia, comprising administering antithrombin to a preeclampsia patient having blood antithrombin activity of 100% or less.
2. The method for treating preeclampsia described in 1 above, comprising selecting a preeclampsia patient having blood antithrombin activity of 100% or less as a subject to which antithrombin is administered.
3. A method for treating preeclampsia, comprising administering antithrombin to a preeclampsia patient having systolic blood pressure of 160 mmHg or more or diastolic blood pressure of 110 mmHg or more.
4. The method for treating preeclampsia described in 3 above, comprising selecting a preeclampsia patient having systolic blood pressure of 160 mmHg or more or diastolic blood pressure of 110 mmHg or more as a subject to which antithrombin is administered.
5. A method for treating preeclampsia, comprising administering antithrombin to a preeclampsia patient having blood antithrombin activity of 100% or less and having systolic blood pressure of 160 mmHg or more or diastolic blood pressure of 110 mmHg or more.
6. The method for treating preeclampsia described in 5 above, comprising selecting a preeclampsia patient having blood antithrombin activity of 100% or less and having systolic blood pressure of 160 mmHg or more or diastolic blood pressure of 110 mmHg or more as a subject to which antithrombin is administered.
7. The method for treating preeclampsia described in any one of 1 to 6 above, wherein the preeclampsia is early-onset preeclampsia.
8. The method for treating preeclampsia described in any one of 1 to 7 above, wherein the antithrombin is human antithrombin.
9. The method for treating preeclampsia described in 8 above, wherein the human antithrombin is recombinant human antithrombin or plasma-derived human antithrombin.
10. The method for treating preeclampsia described in 9 above, wherein the recombinant human antithrombin is antithrombin gamma.
11. A composition for treating preeclampsia comprising antithrombin, which is administered to a preeclampsia patient having blood antithrombin activity of 100% or less.
12. The composition for treating preeclampsia described in 11 above, comprising selecting a preeclampsia patient having blood antithrombin activity of 100% or less as a subject to which antithrombin is administered.
13. A composition for treating preeclampsia comprising antithrombin, which is administered to a preeclampsia patient having systolic blood pressure of 160 mmHg or more or diastolic blood pressure of 110 mmHg or more.
14. The composition for treating preeclampsia described in 13 above, comprising selecting a preeclampsia patient having systolic blood pressure of 160 mmHg or more or diastolic blood pressure of 110 mmHg or more as a subject to which antithrombin is administered.
15. A composition for treating preeclampsia comprising antithrombin, which is administered to a preeclampsia patient having blood antithrombin activity of 100% or less and having systolic blood pressure of 160 mmHg or more or diastolic blood pressure of 110 mmHg or more.
16. The composition for treating preeclampsia described in 15 above, comprising selecting a preeclampsia patient having blood antithrombin activity of 100% or less and having systolic blood pressure of 160 mmHg or more or diastolic blood pressure of 110 mmHg or more as a subject to which antithrombin is administered.
17. The composition for treating preeclampsia described in any one of 11 to 16 above, wherein the preeclampsia is early-onset preeclampsia.
18. The composition for treating preeclampsia described in any one of 11 to 17 above, wherein the antithrombin is human antithrombin.
19. The composition for treating preeclampsia described in 18 above, wherein the human antithrombin is recombinant human antithrombin or plasma-derived human antithrombin.
20. The composition for treating preeclampsia described in 19 above, wherein the recombinant human antithrombin is antithrombin gamma.

### Effects of the Invention

The method for treating PE and the composition for treating PE of the invention extend the pregnancy duration of a patient. Extension of the pregnancy duration of a PE patient leads to the growth of the fetus and is expected to reduce neonatal death and complications such as cerebral palsy.

According to the invention, a method for treating preeclampsia or a composition for treating preeclampsia can be provided.

### Brief Description of Drawings

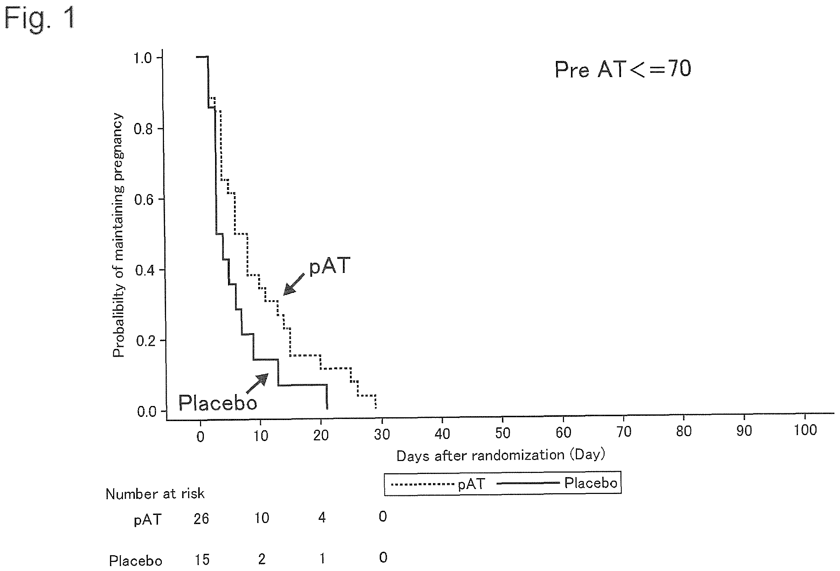

[Fig. 1] Fig. 1 is a figure showing a Kaplan-Meier plot of the continued pregnancy rates and the pregnancy duration (day) after human plasma-derived antithrombin administration and placebo administration of patient groups having PreAT activity of 70% or less. The vertical axis shows the continued pregnancy rate, and the horizontal axis shows the pregnancy duration (day). In the figure, pAT means human plasma-derived antithrombin.

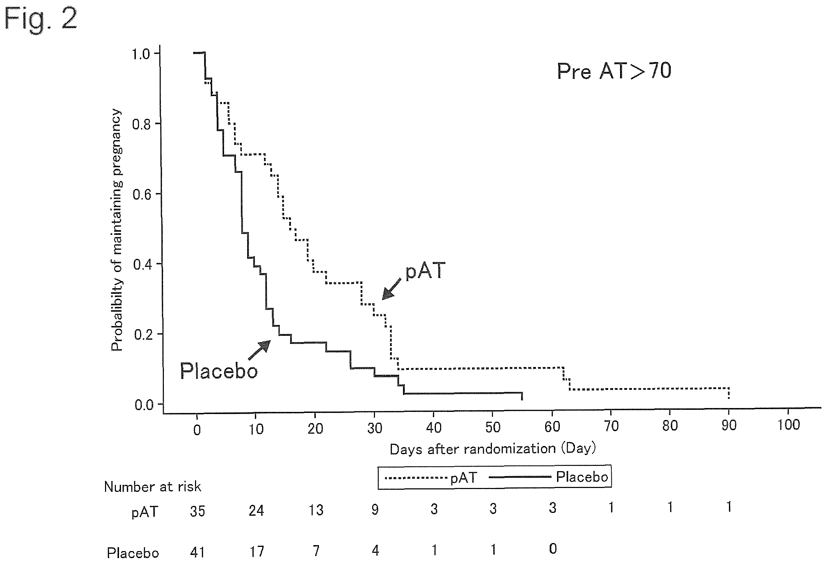

[Fig. 2] Fig. 2 is a figure showing a Kaplan-Meier plot of the continued pregnancy rates and the pregnancy duration (day) after human plasma-derived antithrombin administration and placebo administration of patient groups having PreAT activity exceeding 70%. The vertical axis shows the continued pregnancy rate, and the horizontal axis shows the pregnancy duration (day). In the figure, pAT means human plasma-derived antithrombin.

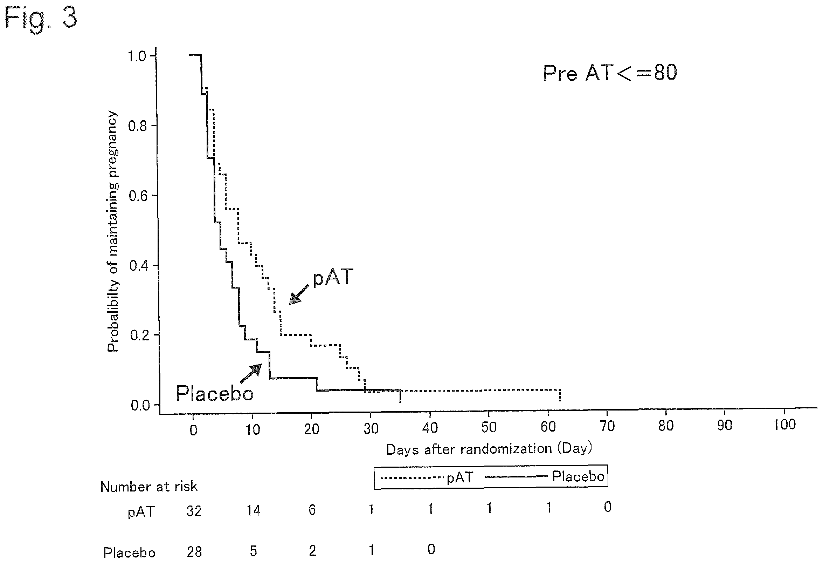

[Fig. 3] Fig. 3 is a figure showing a Kaplan-Meier plot of the continued pregnancy rates and the pregnancy duration (day) after human plasma-derived antithrombin administration and placebo administration of patient groups having PreAT activity of 80% or less. The vertical axis shows the continued pregnancy rate, and the horizontal axis shows the pregnancy duration (day). In the figure, pAT means human plasma-derived antithrombin.

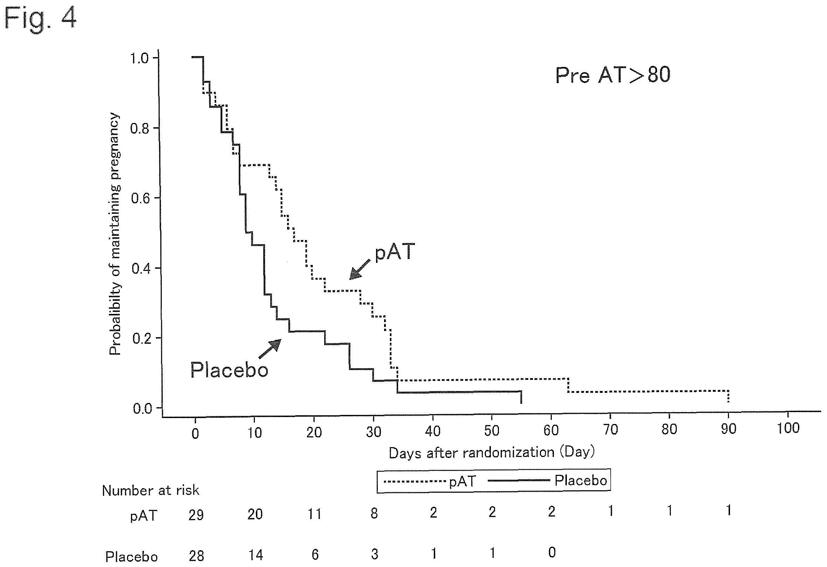

[Fig. 4] Fig. 4 is a figure showing a Kaplan-Meier plot of the continued pregnancy rates and the pregnancy duration (day) after human plasma-derived antithrombin administration and placebo administration of patient groups having PreAT activity exceeding 80%. The vertical axis shows the continued pregnancy rate, and the horizontal axis shows the pregnancy duration (day). In the figure, pAT means human plasma-derived antithrombin.

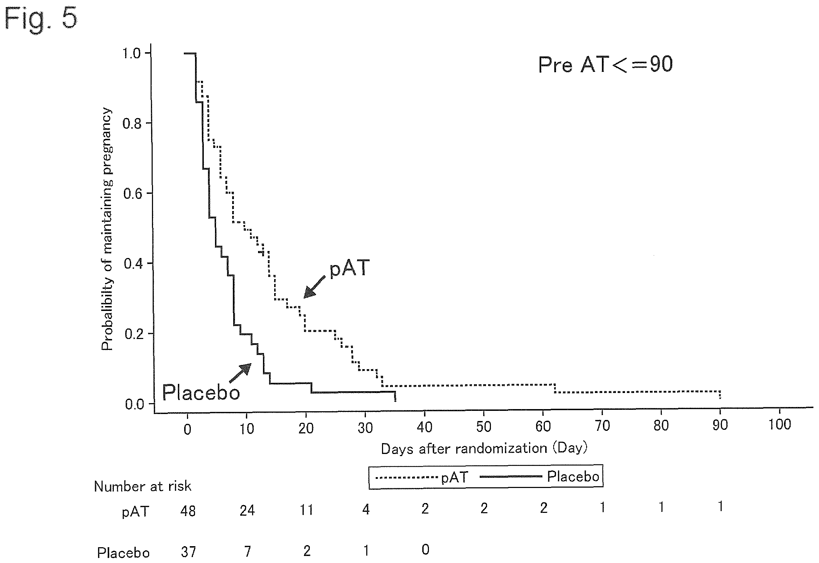

[Fig. 5] Fig. 5 is a figure showing a Kaplan-Meier plot of the continued pregnancy rates and the pregnancy duration (day) after human plasma-derived antithrombin administration and placebo administration of patient groups having PreAT activity of 90% or less. The vertical axis shows the continued pregnancy rate, and the horizontal axis shows the pregnancy duration (day). In the figure, pAT means human plasma-derived antithrombin.

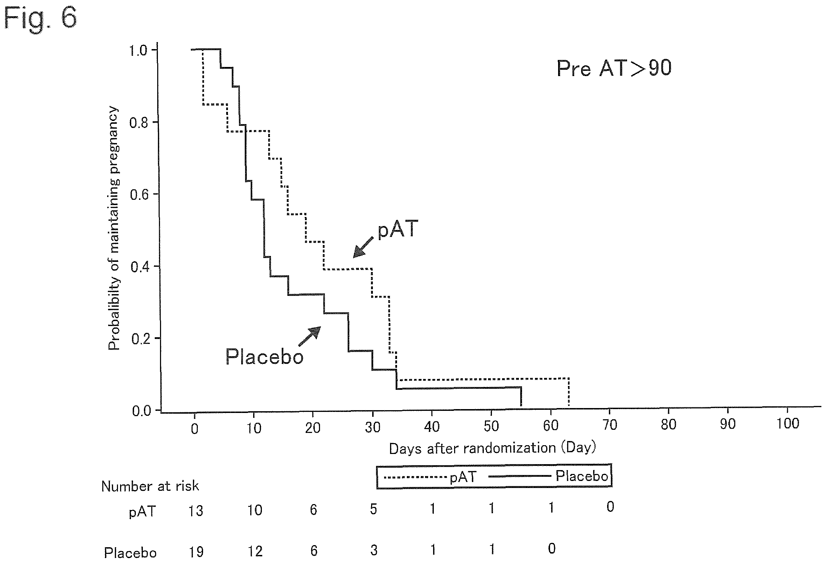

[Fig. 6] Fig. 6 is a figure showing a Kaplan-Meier plot of the continued pregnancy rates and the pregnancy duration (day) after human plasma-derived antithrombin administration and placebo administration of patient groups having PreAT activity exceeding 90%. The vertical axis shows the continued pregnancy rate, and the horizontal axis shows the pregnancy duration (day). In the figure, pAT means human plasma-derived antithrombin.

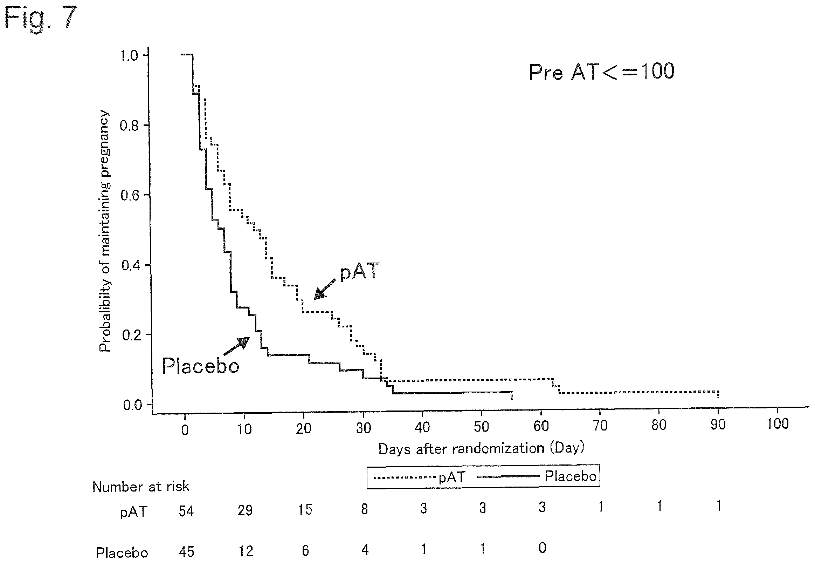

[Fig. 7] Fig. 7 is a figure showing a Kaplan-Meier plot of the continued pregnancy rates and the pregnancy duration (day) after human plasma-derived antithrombin administration and placebo administration of patient groups having PreAT activity of 100% or less. The vertical axis shows the continued pregnancy rate, and the horizontal axis shows the pregnancy duration (day). In the figure, pAT means human plasma-derived antithrombin.

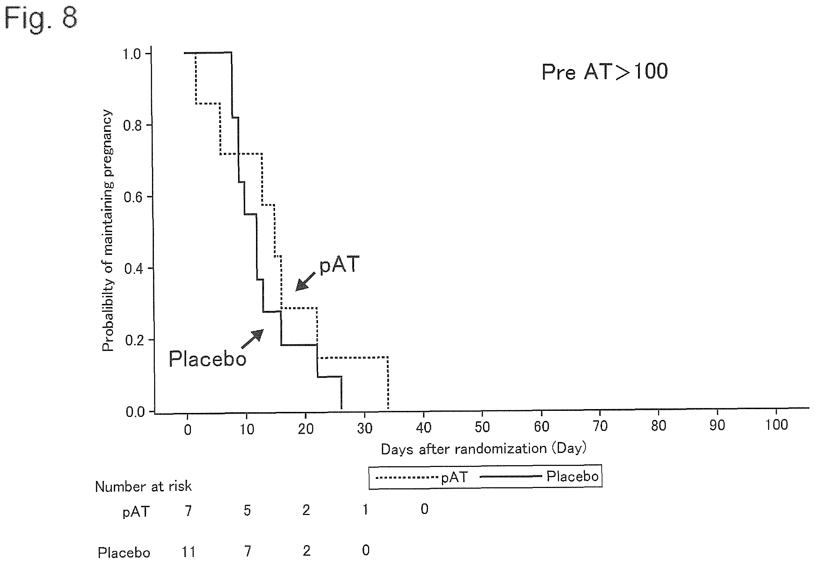

[Fig. 8] Fig. 8 is a figure showing a Kaplan-Meier plot of the continued pregnancy rates and the pregnancy duration (day) after human plasma-derived antithrombin administration and placebo administration of patient groups having PreAT activity exceeding 100%. The vertical axis shows the continued pregnancy rate, and the horizontal axis shows the pregnancy duration (day). In the figure, pAT means human plasma-derived antithrombin.

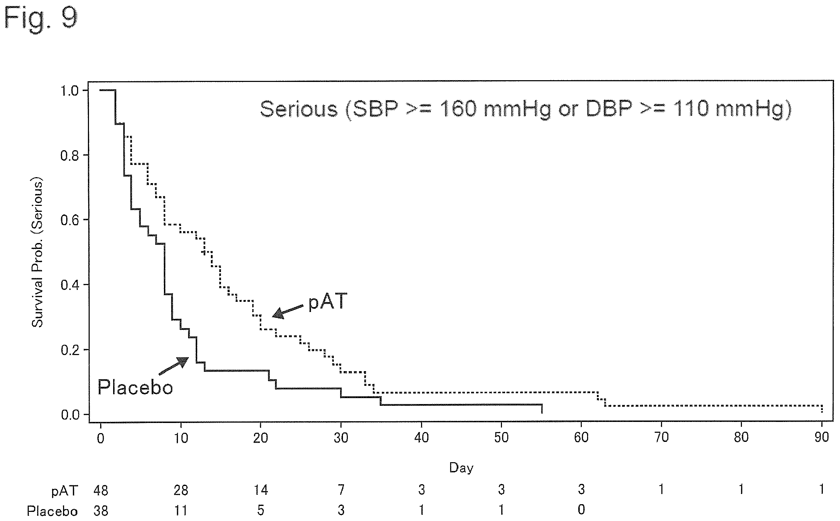

[Fig. 9] Fig. 9 is a figure showing a Kaplan-Meier plot of the continued pregnancy rates and the pregnancy duration (day) after human plasma-derived antithrombin administration and placebo administration of serious patient groups having diastolic blood pressure (referred to as SBP below) of 160 mmHg or more before the drug administration or diastolic blood pressure (referred to as DBP below) of 110 mmHg or more before the drug administration. The vertical axis shows the continued pregnancy rate, and the horizontal axis shows the pregnancy duration (day). In the figure, pAT means human plasma-derived antithrombin.

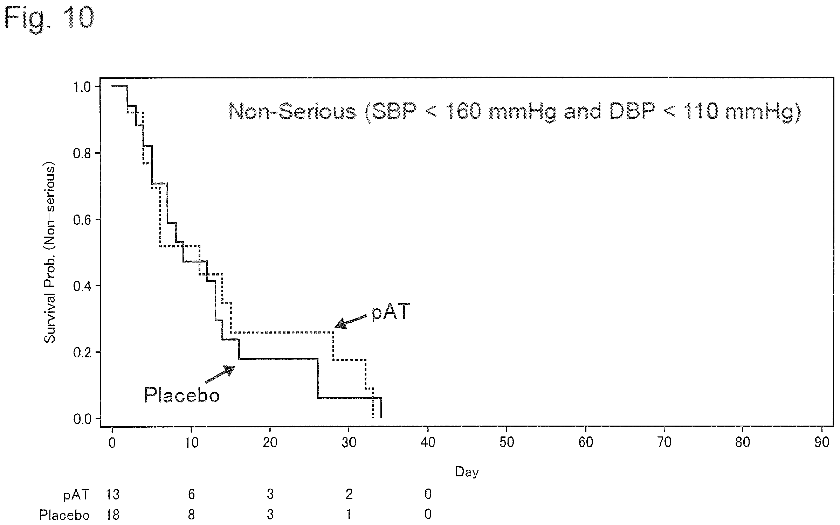

[Fig. 10] Fig. 10 is a figure showing a Kaplan-Meier plot of the continued pregnancy rates and the pregnancy duration (day) after human plasma-derived antithrombin administration and placebo administration of non-serious patient groups having SBP of less than 160 mmHg before the drug administration or DBP of less than 110 mmHg before the drug administration. The vertical axis shows the continued pregnancy rate, and the horizontal axis shows the pregnancy duration (day). In the figure, pAT means human plasma-derived antithrombin.

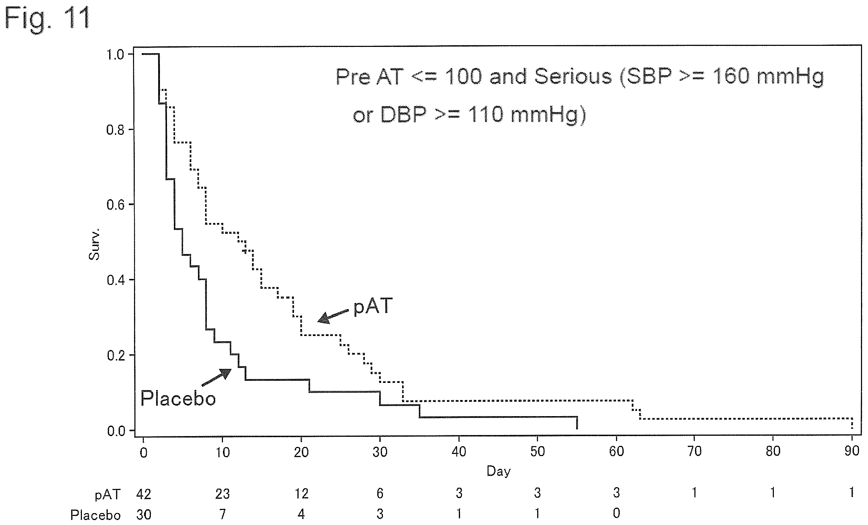

[Fig. 11] Fig. 11 is a figure showing a Kaplan-Meier plot of the continued pregnancy rates and the pregnancy duration (day) after human plasma-derived antithrombin administration and placebo administration of serious patient groups having PreAT activity of 100% or less and having SBP of 160 mmHg or more before the drug administration or DBP of 110 mmHg or more before the drug administration. The vertical axis shows the continued pregnancy rate, and the horizontal axis shows the pregnancy duration (day). In the figure, pAT means human plasma-derived antithrombin.

### Embodiments for Carrying Out the Invention

The invention is explained in detail below.

Preeclampsia (referred to as PE below) is a disease type of hypertensive disorders of pregnancy (referred to as HDP below), in which hypertension is observed during pregnancy.

PE is a condition in which hypertension first develops at 20 weeks of pregnancy or later accompanied by proteinuria and the condition becomes normal before 12 weeks after the delivery, or even when proteinuria is not observed, liver dysfunction, renal dysfunction, neuropathy, blood coagulation disorder or uteroplacental insufficiency is observed.

Hypertension refers to a case in which the systolic blood pressure is 140 mmHg or more or in which the diastolic blood pressure is 90 mmHg or more.

Proteinuria refers to a case in which 300 mg/day or more of protein is detected from 24-hour urine by the Esbach method or the like or a case of a protein/creatinine (P/C) ratio of 0.3 mg/mg·CRE or more in a random urine sample.

PE is defined as serious when the systolic blood pressure is 160 mmHg or more or the diastolic blood pressure is 110 mmHg or more or when organ dysfunction of the mother or uteroplacental insufficiency is observed.

PE includes early onset type, in which the onset is at 20 weeks of pregnancy or later but before 34 weeks, and late onset type, in which the onset is at 34 weeks of pregnancy or later.

In the invention, the therapeutic effects on PE can be assessed, for example, by the pregnancy duration of a patient or the like.

Antithrombin is also called antithrombin III. In the invention, antithrombin is preferably antithrombin of human (referred to as human antithrombin below) but is not particularly limited thereto.

In human, antithrombin is a physiological serine protease inhibitor which is produced in the liver and which regulates coagulation as a coagulation inhibitor in the blood. Antithrombin binds to and inactivates coagulation factors such as thrombin (activated Factor II), activated Factor X (Factor Xa), Factor IX (Factor IXa) and kallikrein and fibrinolytic plasmin. Thrombin generated by activation of the coagulation system immediately forms a complex with antithrombin and is inactivated. Moreover, antithrombin enhances the antithrombotic activity through binding of heparin.

In the invention, the human antithrombin is recombinant human antithrombin or human antithrombin derived from human plasma (referred to as plasma-derived human antithrombin below). Specific examples of the recombinant human antithrombin include antithrombin gamma, recombinant human antithrombins described in WO2005/035563 and WO2008/120801 and the like. An example of the plasma-derived human antithrombin is dry concentrated human antithrombin III, and more specific examples include Anthrobin P (registered trademark), Kenketu Nonthron (registered trademark), Neuart (registered trademark) and the like. Other specific examples of the plasma-derived human antithrombin include Thrombate III, Kedrion, Atenativ and the like.

The antithrombin activity is the specific activity of human antithrombin quantified by adding heparin and thrombin to the target human antithrombin to form complexes, then adding a substrate and measuring the absorbance of the chromogenic compound released from the substrate due to thrombin remaining dependently on the human antithrombin concentration.

The antithrombin activity is one of the blood coagulation/fibrinolysis tests in human, and the standard value is 80 to about 120% or 80 to 130%.

The antithrombin activity value can be measured with a commercial blood testing antithrombin III kit for measurement using plasma, whole blood or the like (Pharmaceutical and Food Safety Bureau Notice 0329, No. 10, "Notice on Changes to General Designations for Extracorporeal Diagnostic Drugs", March 29, 2011, director of Pharmaceutical and Food Safety Bureau, Ministry of Health, Labour and Welfare) or the like such as TriniCHROM (registered trademark) ATXa (manufactured by Kyowa Medex Co., Ltd.), L System ATIII, Berichrom Antithrombin III Automatic B and L System ATIII (manufactured by Sysmex Corporation), Test Team (registered trademark) Neo ATIII, Test Team (registered trademark) ATIII 2 Kit and Test Team (registered trademark) S ATIII (manufactured by Sekisui Medical Co., Ltd.), Evatest ATIII (manufactured by Nissui Pharmaceutical Co., Ltd.), ATIII Liquid and STA reagent series (manufactured by Roche Diagnostics K.K.), N-Assay TIA ATIII, N-Assay L ATIII Nittobo (manufactured by Nittobo Medical Co., Ltd.) and Chromorate ATIII(C) II (manufactured by LSI Medience Corporation).

An example of the antithrombin activity value is the blood antithrombin activity value.

In the invention, examples of blood antithrombin activity of 100% or less include blood antithrombin activity of preferably 100 to 90%, 100 to 80%, 100 to 70%, 100 to 60%, 100 to 50%, 100 to 40%, 100 to 30%, 100 to 20%, 100 to 10% or 100 to 0% and the like.

In the invention, examples of blood antithrombin activity of 100% or less also include blood antithrombin activity of 100% or less, 90% or less, 80% or less, 70% or less, 60% or less, 50% or less, 40% or less, 30% or less, 20% or less or 10% or less and the like.

The therapeutic effects of the therapeutic method and the therapeutic composition of the invention can be assessed, for example, by extension of the pregnancy duration or the like of patients to whom antithrombin or the therapeutic composition containing antithrombin has been administered as compared to that of patients to whom a placebo has been administered.

Examples of the daily dosage of antithrombin or the therapeutic composition containing antithrombin in the invention include 72 IU/kg in the case of antithrombin gamma, 3000 IU in the case of Anthrobin P and the like.

Antithrombin or the therapeutic composition containing antithrombin in the invention may be administered once or administered multiple times a day. Administration of once or multiple times a day may be continued for multiple days. In the case of administration for multiple days, administration may be conducted every day or once in some days, but administration is preferably conducted every day.

Examples for the case in which the antithrombin is recombinant human antithrombin include administration of 72 IU/kg once a day every day for seven days and the like.

When antithrombin or the therapeutic composition containing antithrombin is administered multiple times a day or administered for multiple days, the blood antithrombin activity of the patient of 100% or less and/or the systolic blood pressure of 160 mmHg or more or the diastolic blood pressure of 110 mmHg or more may be the values before the administration of any administration time but are preferably the values before the first administration.

The method for administering antithrombin or the therapeutic composition containing antithrombin in the invention is IV injection (also referred to as intravenous administration or intravenous injection) or IV infusion (also referred to as intravenous infusion or infusion).

The therapeutic composition containing antithrombin in the invention is preferably provided as a composition produced by any well-known method in the technical field of drug formulation by mixing with one or more pharmacologically generally acceptable carriers, additives, pH regulators or the like for the use as a pharmaceutical preparation.

The therapeutic composition containing antithrombin can be prepared as a solution-type injection using a carrier composed of an amino acid, sugar, a salt, a buffer, a mixture thereof or the like, an additive, a pH regulator or the like as a suitable composition for IV injection or IV infusion.

Alternatively, the therapeutic composition containing antithrombin can be prepared as a powdery injection obtained by freeze-drying antithrombin or a therapeutic composition containing antithrombin according to a normal method. In the case of administration as a powdery injection, the powder containing antithrombin or the therapeutic composition containing antithrombin is dissolved in advance in water for injection or a solution such as physiological saline and then used.

The therapeutic composition containing antithrombin is preferably a composition containing sodium citrate, sodium citrate hydrate, glycine, sodium L-glutamate, D-mannitol or sodium chloride in addition to antithrombin, more preferably a composition containing glycine and sodium citrate hydrate or a composition containing glycine, sodium citrate hydrate and sodium chloride, further preferably a composition containing glycine, sodium citrate hydrate, sodium chloride, hydrochloric acid and sodium hydroxide. Such a composition may contain an appropriate surfactant. The surfactant is polysorbate 20, polysorbate 80 or the like.

The rate for administering antithrombin or the therapeutic composition containing antithrombin to a patient through IV injection or IV infusion in the invention is not particularly limited, but an example is slow administration.

### Examples

The invention is explained more specifically by Examples below, but the Examples are mere examples of the invention and do not limit the scope of the invention.

### [Example 1]

The data obtained in the following clinical trials conducted between July 1993 and August 1995 were analyzed in the Examples below.

Subjects: pure serious pregnancy toxemia patients having total Gestosis Index scores of 6 or more (corresponding to PE according to the current diagnostic standard)

Trial Method:
(1) Trial Design: a telephone-based, placebo-controlled, multicenter, double-blind, randomized, parallel-group trial using minimization method
(2) Study Drug: plasma-derived human antithrombin formulation
(3) Control Drug: placebo (albumin) formulation
(4) Dosage and Administration Method: Six vials (3000U) of the plasma-derived human antithrombin formulation were intravenously administered once a day, and six vials (582 mg) of the placebo formulation were intravenously administered once a day.
(5) Trial Period: Total 14 days including 7 continuous days of administration and 7 days of post-treatment observation period

### [Example 2]

The pregnancy duration data of the patients obtained in the clinical trials described in Example 1 were analyzed by subgroup analysis using the blood antithrombin (Pre AT) activity of the patients before the drug administration (Day0, before the first administration) as the index.

The results of a Kaplan-Meier plot of the continued pregnancy rates and the pregnancy duration (day) after the human antithrombin administration and the placebo administration of the patient groups having Pre AT activity of 70% or less, exceeding 70%, 80% or less, exceeding 80%, 90% or less, exceeding 90%, 100% or less or exceeding 100% are shown in Fig. 1 to Fig. 8, respectively.

Moreover, the numbers N (subjects) of the patient groups and the means (Means) and the standard deviations (SE) of the pregnancy duration calculated by the Kaplan-Meier method are shown in Table 1.

**[Table 1]**

| Pre AT Activity (%) | Administration Group | N (subjects) | Mean (SE) (Day) of Pregnancy Duration |
|---|---|---|---|
| Pre AT <= 70 | Antithrombin administration | 26 | 9.8 (1.5) |
| | Placebo administration | 15 | 6.0 (1.4) |
| Pre AT > 70 | Antithrombin administration | 35 | 21.8 (3.4) |
| | Placebo administration | 41 | 12.0 (1.7) |
| Pre AT <= 80 | Antithrombin administration | 32 | 12.2 (2.2) |
| | Placebo administration | 28 | 7.3 (1.4) |
| Pre AT > 80 | Antithrombin administration | 29 | 21.4 (3.6) |
| | Placebo administration | 28 | 13.6 (2.2) |
| Pre AT <= 90 | Antithrombin administration | 48 | 15.0 (2.4) |
| | Placebo administration | 37 | 7.1 (1.1) |
| Pre AT > 90 | Antithrombin administration | 13 | 22.2 (4.6) |
| | Placebo administration | 19 | 17.0 (2.9) |
| Pre AT <= 100 | Antithrombin administration | 54 | 16.8 (2.4) |
| | Placebo administration | 45 | 9.8 (1.6) |
| Pre AT > 100 | Antithrombin administration | 7 | 15.4 (4.0) |
| | Placebo administration | 11 | 13.2 (1.8) |

From Table 1, no extension of the pregnancy duration was observed by the antithrombin administration as compared to the placebo administration in the patient group having Pre AT activity exceeding 100%. On the other hand, in all the patient groups having Pre AT activity of 100% or less, extension of the pregnancy duration was observed by the antithrombin administration as compared to the placebo administration.

### [Example 3]

The pregnancy duration data of the patients obtained in the trials described in Example 1 were analyzed by subgroup analysis using the systolic blood pressure (SBP) of the patients before the drug administration (Day0, before the first administration) or the diastolic blood pressure (DBP) of the patients before the drug administration as the index.

A Kaplan-Meier plot of the continued pregnancy rates and the pregnancy duration (day) after the human antithrombin administration and the placebo administration of the serious patient groups having SBP of 160 mmHg or more before the administration of human antithrombin or albumin or DBP of 110 mmHg or more before the administration of human antithrombin or albumin was obtained. The results obtained are shown in Fig. 9.

A Kaplan-Meier plot of the continued pregnancy rates and the pregnancy duration (day) after the human antithrombin administration and the placebo administration of the non-serious patient groups having SBP of less than 160 mmHg before the administration of human antithrombin or albumin and DBP of less than 110 mmHg before the administration of human antithrombin or albumin was obtained. The results obtained are shown in Fig. 10.

Moreover, the numbers N (subjects) of the patient groups and the means (Means) and the standard deviations (SE) of the pregnancy duration calculated by the Kaplan-Meier method are shown in Table 2.

**[Table 2]**

| | Administration Group | N (subjects) | Mean (SE) (Day) of Pregnancy Duration |
|---|---|---|---|
| Serious (SBP >= 160 mmHg or DBP >= 110 mmHg) | Antithrombin administration | 48 | 17.3 (2.6) |
| | Placebo administration | 38 | 9.8 (1.7) |
| Non-serious (SBP < 160 mmHg and DBP < 110 mmHg) | Antithrombin administration | 13 | 13.7 (3.3) |
| | Placebo administration | 18 | 12.0 (2.2) |

From Table 2, no extension of the pregnancy duration was observed by the antithrombin administration as compared to the placebo administration in the non-serious patient group having SBP of less than 160 mmHg before the drug administration and DBP of less than 110 mmHg before the drug administration.

On the other hand, extension of the pregnancy duration was observed by the antithrombin administration as compared to the placebo administration in the serious patient group having SBP of 160 mmHg or more before the drug administration or DBP of 110 mmHg or more before the drug administration.

### [Example 4]

The pregnancy duration data of the patients obtained in the clinical trials described in Example 1 were analyzed by subgroup analysis using the blood antithrombin activity of the patients before the drug administration and the SBP of the patients before the drug administration or the DBP of the patients before the drug administration as the indexes. "Before the drug administration" in each case means Day0, namely before the first administration.

The results of a Kaplan-Meier plot of the continued pregnancy rates and the pregnancy duration (day) after the human antithrombin administration and the placebo administration of the serious patient groups having blood antithrombin activity (Pre AT) of 100% or less before the administration of human antithrombin or albumin and having SBP of 160 mmHg or more before the administration of human antithrombin or albumin or DBP of 110 mmHg or more before the administration of human antithrombin or albumin are shown in Fig. 11.

The numbers N (subjects) of the patient groups and the means (Means) and the standard deviations (SE) of the pregnancy duration calculated by the Kaplan-Meier method are shown in Table 3.

**[Table 3]**

| | Administration Group | N (subjects) | Mean (SE) (Day) of Pregnancy Duration |
|---|---|---|---|
| Pre AT <= 100 and serious (SBP >= 160 mmHg or DBP >= 110 mmHg) | Antithrombin administration | 42 | 17.4 (2.9) |
| | Placebo administration | 30 | 9.4 (2.1) |

From Table 3, extension of the pregnancy duration was observed by the antithrombin administration as compared to the placebo administration in the serious patient group having blood antithrombin activity of 100% or less before the drug administration and having SBP of 160 mmHg or more before the drug administration or DBP of 110 mmHg or more before the drug administration.

### [Example 5]

With respect to the results of the subgroup analyses obtained in Examples 2 to 4, the numbers N (subjects) of the patient groups 1) to 4) below and the means (Means) and the standard deviations (SE) of the pregnancy duration after the human antithrombin administration and the placebo administration calculated by the Kaplan-Meier method are shown in Table 4.

1) All patients (GI value of 6 or more),
2) patient group having blood antithrombin (Pre AT) activity of 100% or less before the administration of human antithrombin or albumin,
3) serious patient group having SBP of 160 mmHg or more before the administration of human antithrombin or albumin or DBP of 110 mmHg or more before the administration of human antithrombin or albumin, and
4) serious patient group having blood antithrombin activity (Pre AT) of 100% or less before the administration of human antithrombin or albumin and having SBP of 160 mmHg or more before the administration of human antithrombin or albumin or DBP of 110 mmHg or more before the administration of human antithrombin or albumin.

**[Table 4]**

| | Mean ± SE (Day) of Pregnancy Duration (N (subjects)) | | Extension of Pregnancy Duration of AT Administration Group Compared to Placebo Administration Group (Day) |
|---|---|---|---|
| | AT Administration | Placebo Administration | |
| GI value of 6 or more | 16.8 ± 2.0 (66) | 10.2 ± 1.2 (67) | 6.6 |
| Pre AT <= 100 | 16.8 ± 2.4 (54) | 9.8 ± 1.6 (45) | 7.0 |
| Serious (SBP >= 160 mmHg or DBP >= 110 mmHg) | 17.3 ± 2.6 (48) | 9.8 ± 1.7 (38) | 7.5 |
| Pre AT <= 100 and serious (SBP >= 160 mmHg or DBP >= 110 mmHg) | 17.4 ± 2.9 (42) | 9.4 ± 2.1 (30) | 8.0 |

As shown in Table 4, extension of the pregnancy duration was observed by the antithrombin administration as compared to the placebo administration in all the patient groups as compared to all the patients.

In particular, the longest extension of the pregnancy duration was observed by the antithrombin administration as compared to the placebo administration in the serious patient group having blood antithrombin activity of 100% or less before the drug administration and having SBP of 160 mmHg or more before the drug administration or DBP of 110 mmHg or more before the drug administration.

Although the invention has been explained in detail using specific embodiments, it is obvious to one skilled in the art that various changes and modifications can be made without departing from the intension and the scope of the invention. This application is based on a U.S. provisional application filed on February 11, 2019 (US62/803,888) and a U.S. provisional application filed on March 19, 2019 (US62/820,459), which are hereby incorporated by reference in their entirety.

## Claims

1. A method for treating preeclampsia, comprising administering antithrombin to a preeclampsia patient having blood antithrombin activity of 100% or less.

2. The method for treating preeclampsia according to claim 1, comprising selecting a preeclampsia patient having blood antithrombin activity of 100% or less as a subject to which antithrombin is administered.

3. A method for treating preeclampsia, comprising administering antithrombin to a preeclampsia patient having systolic blood pressure of 160 mmHg or more or diastolic blood pressure of 110 mmHg or more.

4. The method for treating preeclampsia according to claim 3, comprising selecting a preeclampsia patient having systolic blood pressure of 160 mmHg or more or diastolic blood pressure of 110 mmHg or more as a subject to which antithrombin is administered.

5. A method for treating preeclampsia, comprising administering antithrombin to a preeclampsia patient having blood antithrombin activity of 100% or less and having systolic blood pressure of 160 mmHg or more or diastolic blood pressure of 110 mmHg or more.

6. The method for treating preeclampsia according to claim 5, comprising selecting a preeclampsia patient having blood antithrombin activity of 100% or less and having systolic blood pressure of 160 mmHg or more or diastolic blood pressure of 110 mmHg or more as a subject to which antithrombin is administered.

7. The method for treating preeclampsia according to any one of claims 1 to 6, wherein the preeclampsia is early-onset preeclampsia.

8. The method for treating preeclampsia according to any one of claims 1 to 7, wherein the antithrombin is human antithrombin.

9. The method for treating preeclampsia according to claim 8, wherein the human antithrombin is recombinant human antithrombin or plasma-derived human antithrombin.

10. The method for treating preeclampsia according to claim 9, wherein the recombinant human antithrombin is antithrombin gamma.

11. A composition for treating preeclampsia comprising antithrombin, which is administered to a preeclampsia patient having blood antithrombin activity of 100% or less.

12. The composition for treating preeclampsia according to claim 11, comprising selecting a preeclampsia patient having blood antithrombin activity of 100% or less as a subject to which antithrombin is administered.

13. A composition for treating preeclampsia, comprising antithrombin, which is administered to a preeclampsia patient having systolic blood pressure of 160 mmHg or more or diastolic blood pressure of 110 mmHg or more.

14. The composition for treating preeclampsia according to claim 13, comprising selecting a preeclampsia patient having systolic blood pressure of 160 mmHg or more or diastolic blood pressure of 110 mmHg or more as a subject to which antithrombin is administered.

15. A composition for treating preeclampsia comprising antithrombin, which is administered to a preeclampsia patient having blood antithrombin activity of 100% or less and having systolic blood pressure of 160 mmHg or more or diastolic blood pressure of 110 mmHg or more.

16. The composition for treating preeclampsia according to claim 15, comprising selecting a preeclampsia patient having blood antithrombin activity of 100% or less and having systolic blood pressure of 160 mmHg or more or diastolic blood pressure of 110 mmHg or more as a subject to which antithrombin is administered.

17. The composition for treating preeclampsia according to any one of claims 11 to 16, wherein the preeclampsia is early-onset preeclampsia.

18. The composition for treating preeclampsia according to any one of claims 11 to 17, wherein the antithrombin is human antithrombin.

19. The composition for treating preeclampsia according to claim 18, wherein the human antithrombin is recombinant human antithrombin or plasma-derived human antithrombin.

20. The composition for treating preeclampsia according to claim 19, wherein the recombinant human antithrombin is antithrombin gamma.
